# EUROPEAN PATENT SPECIFICATION

(11) **EP 4 110 213 B1**
(45) Date of publication and mention of the grant of the patent: **21.08.2024**
(21) Application number: 21710845.5
(22) Date of filing: 18.02.2021
(51) Int. Cl.: A61B 18/24, A61B 18/26, A61B 17/22, A61B 18/04, A61B 18/28, A61B 18/00, A61B 90/00

(54) **FLUID RECIRCULATION SYSTEM FOR INTRAVASCULAR LITHOTRIPSY DEVICE**
FLÜSSIGKEITSREZIRKULATIONSSYSTEM FÜR EINE INTRAVASKULÄRE LITHOTRIPSIEVORRICHTUNG
SYSTÈME DE RECIRCULATION DE FLUIDE POUR UN DISPOSITIF DE LITHOTRITIE INTRAVASCULAIRE

(30) Priority: 27.02.2020 US 202062982672 P; 17.02.2021 US 202117178184
(43) Date of publication of application: 04.01.2023
(73) Proprietor: Bolt Medical, Inc., Carlsbad, CA 92008 (US)
(72) Inventor: SCHULTHEIS, Eric A., San Clemente, CA 92673 (US); COOK, Christopher A., Laguna Niguel, CA 92677 (US)
(74) Representative: Potter Clarkson
(86) International application number: PCT/US2021/018522
(87) International publication number: WO 2021/173417

(56) References cited:
- DE-A1- 3 038 445
- US-A1- 2011 257 641
- US-A1- 2012 071 715
- US-A1- 2016 016 016
- US-A1- 2018 360 482

## Description

### RELATED APPLICATIONS

This application claims priority on United States Provisional Application Serial No. 62/982,672, filed on February 27, 2020, and on United States Patent Application Serial No. 17/178,184, filed on February 17, 2021. As far as permitted, the contents of United States Provisional Application Serial No. 62/982,672 and United States Patent Application Serial No. 17/178,184.

### BACKGROUND

Vascular lesions within vessels in the body can be associated with an increased risk for major adverse events, such as myocardial infarction, embolism, deep vein thrombosis, stroke, and the like. Severe vascular lesions can be difficult to treat and achieve patency for a physician in a clinical setting.

Vascular lesions may be treated using interventions such as drug therapy, balloon angioplasty, atherectomy, stent placement, vascular graft bypass, to name a few. Such interventions may not always be ideal or may require subsequent treatment to address the lesion.

Intravascular lithotripsy (IVL) is one method that has been recently used with some success for breaking up vascular lesions within vessels in the body. During an IVL treatment, a high energy source, e.g., a high energy laser, is used to generate plasma and ultimately shockwaves within a fluid-filled balloon to crack calcification at a lesion site within the vasculature. As the high energy applications are delivered to the fluid within the balloon, the fluid within the balloon can heat up such that the fluid experiences temperature increases above safe levels. Thus, it is desired to provide a system whereby the temperature of the fluid within the balloon can be effectively controlled so that it does not increase above safe levels. For example, in certain applications, it is desirable to maintain the temperature of the fluid within the balloon at approximately 37ºC throughout the procedure.

Additionally, the high energy applications delivered to the fluid within the balloon can also hit a plasma target or other structures, which can cause microparticles to get into the fluid within the balloon. Such microparticles in the fluid can cause the plasma to be less concentrated and more distributed, which can have an adverse impact on the performance of the IVL device. Accordingly, it is further desired to provide a system that can effectively minimize such microparticles that may otherwise be present within the fluid within the balloon.

### SUMMARY

The invention is defined in claim 1. Further embodiments are defined in the dependent claims. No surgical methods form part of the invention.

The present invention is directed toward a catheter system for placement within a blood vessel having a vessel wall. The catheter system can be used for treating a treatment site within or adjacent to the vessel wall or heart valve. In various embodiments, the catheter system includes a balloon and a fluid circulator. The balloon includes a balloon wall that defines a balloon interior. The balloon is configured to retain a catheter fluid within the balloon interior. The fluid circulator can be in fluid communication to the balloon interior. The fluid circulator is configured to selectively circulate the catheter fluid out of and back into the balloon interior during use of the catheter system.

In some embodiments, the fluid circulator is configured so that a temperature of the catheter fluid within the balloon interior is maintained within a predetermined temperature range. Additionally, in certain embodiments, the fluid circulator is configured so that a pressure of the catheter fluid within the balloon interior is maintained within a predetermined pressure range.

In various embodiments, the catheter system further includes a fluid inflow line and a fluid outflow line that are coupled to the fluid circulator. The fluid circulator pumps the catheter fluid out of the balloon interior via the fluid outflow line and pumps the catheter fluid back into the balloon interior via the fluid inflow line. In some such embodiments, the fluid outflow line includes an inlet port that is positioned in the balloon interior so that the catheter fluid can flow out of the balloon interior into the fluid outflow line via the inlet port. Additionally, in certain such embodiments, the fluid inflow line includes an outlet port that is positioned in the balloon interior so that the catheter fluid can flow through the fluid inflow line and back into the balloon interior via the outlet port.

In some embodiments, the catheter system further includes a fluid pump that initially pumps the catheter fluid into the balloon interior to expand the balloon from a collapsed configuration where the balloon is selectively moved relative to the vessel wall, to an expanded configuration suitable for anchoring the balloon in position relative to the vessel wall. Further, when the balloon is in the expanded configuration, the catheter fluid can be within a desired inflation pressure range within the balloon interior. Additionally, in certain embodiments, the fluid pump is selectively in fluid communication with the fluid inflow line via a fluid valve.

In certain embodiments, the catheter system further includes an energy guide that is configured to guide energy into the balloon interior to generate a plasma within the catheter fluid retained within the balloon interior. In such embodiments, the energy guide can include a guide distal end that is positioned within the balloon interior. Additionally, in one embodiment, the energy guide is an optical fiber.

In some embodiments, the catheter system further includes an energy source that directs the energy into and along the energy guide and into the balloon interior. In one embodiment, the energy source includes a laser. In certain such embodiments, the energy source provides sub-millisecond pulses of light energy to the energy guides; and the pulses of light energy are guided along the energy guide and into the balloon interior to generate the plasma within the catheter fluid retained within the balloon interior.

In certain embodiments, the catheter system further includes a fluid temperature sensor that is configured to sense a temperature of the catheter fluid within the balloon interior. Additionally, the fluid temperature sensor can be configured to generate a signal providing an indication of when to selectively activate the fluid circulator. In one embodiment, the fluid temperature sensor is positioned within the balloon interior.

In some embodiments, the catheter system further includes a fluid temperature controller that is configured to control a temperature of the catheter fluid as the catheter fluid is being circulated by the fluid circulator. In one embodiment, the fluid temperature controller includes a chiller. Further, in one embodiment, the fluid temperature controller is positioned adjacent to the fluid circulator.

In certain embodiments, the catheter system further includes a fluid pressure sensor that is configured to sense a pressure of the catheter fluid within the balloon interior. In such embodiments, the fluid pressure sensor can be configured to generate a signal when the pressure of the catheter fluid within the balloon interior falls outside a desired inflation pressure range, the signal providing an indication of when to selectively adjust the fluid circulator to selectively adjust at least one of a first rate at which the circulation fluid is being pumped out of the balloon interior by the fluid circulator and a second rate at which the circulation fluid is being pumped back into the balloon interior by the fluid circulator. Additionally, in one embodiment, the fluid pressure sensor is positioned within the balloon interior.

In some embodiments, the catheter system further includes a fluid pressure controller that is configured to control a pressure of the catheter fluid as the catheter fluid is being circulated by the fluid circulator. In one embodiment, the fluid pressure controller is positioned adjacent to the fluid circulator.

In certain embodiments, the catheter system can include a filtration system that is in fluid communication with the fluid circulator. The filtration system is configured to remove microparticles from the catheter fluid as the catheter fluid is being circulated by the fluid circulator. In some such embodiments, the filtration system includes one or more filters that are configured to remove microparticles from the catheter fluid as the catheter fluid is being circulated by the fluid circulator. Additionally, in certain such embodiments, the catheter fluid is circulated out of the balloon interior and to the fluid circulator along a fluid outflow line; and the filtration system is positioned along the fluid outflow line.

The present disclosure is also directed toward a method for treating a treatment site within or adjacent to a vessel wall or heart valve, the method comprising the steps of providing a balloon including a balloon wall that defines a balloon interior; retaining a catheter fluid within the balloon interior; guiding energy into the balloon interior with an energy guide so that a plasma is generated within the catheter fluid that is retained within the balloon interior; coupling a fluid circulator in fluid communication to the balloon interior; and selectively circulating the catheter fluid out of and back into the balloon interior with the fluid circulator.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims.

US 2018/360482 A1 discloses a shock wave device for treatment of vascular occlusions. First and second conductive wires extend along the length of the device within a volume of the device. A conductive emitter band circumscribes the ends of the first and second wires to form first and second spark gaps between the ends of the first and second wires and the conductive emitter band. The volume is filled with a conductive fluid and a high voltage is applied across the first and second wires to create first and second shock waves initiated from the first and second spark gaps.

US 2012/071715 A1 discloses a method for injecting a therapeutic agent into a target tissue by positioning an expandable member in proximity to the target tissue, introducing the therapeutic agent into the expandable member until the desired pressure is achieved, and then creating a plurality of small apertures/exit sites in the expandable member for the therapeutic agent be administered through.

US 2011/257641 A1 discloses an apparatus for delivering optical energy to a renal artery of a patient comprising a catheter, an optical fiber and an optics arrangement comprising optical elements arranged to receive laser light.

US 2016/016016 A1 discloses a neuromodulation system including a catheter with a balloon along its distal end. Acoustic energy is emitted outward radially by an ultrasound transducer positioned within the interior of the balloon.

DE 30 38 445 A1 discloses a shock wave generator comprising a spark gap for diagnostic or therapeutic applications wherein the generator can be brought in close proximity to the body part being treated with the applied shock waves, in which the spark gap is held at a safe distance from the body tissue and is electrically insulated therefrom.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a schematic cross-sectional view of an embodiment of a catheter system in accordance with various embodiments herein, the catheter system including a fluid recirculation system having features of the present invention; and
Figure 2 is a schematic cross-sectional view of another embodiment of the catheter system including another embodiment of the fluid recirculation system.

While embodiments of the present invention are susceptible to various modifications and alternative forms, specifics thereof have been shown by way of example and drawings, and are described in detail herein. It is understood, however, that the scope herein is not limited to the particular embodiments described. On the contrary, the intention is to cover modifications, equivalents, and alternatives falling within the scope herein.

### DESCRIPTION

Treatment of vascular lesions can reduce major adverse events or death in affected subjects. As referred to herein, a major adverse event is one that can occur anywhere within the body due to the presence of a vascular lesion (also sometime referred to herein as a "treatment site"). Major adverse events can include, but are not limited to, major adverse cardiac events, major adverse events in the peripheral or central vasculature, major adverse events in the brain, major adverse events in the musculature, or major adverse events in any of the internal organs.

The catheter systems and related methods disclosed herein are configured to effectively monitor and/or control a temperature and/or a pressure of a catheter fluid within an inflatable balloon of a IVL catheter. Additionally, the catheter systems and related methods disclosed herein are further configured to filter out and/or remove any undesired microparticles that may be generated or formed within the catheter fluid during use of the catheter system. Further, the catheter systems and related methods disclosed herein can also be configured to remove any undesired air bubbles that may be formed within the catheter fluid within the balloon.

In various embodiments, the catheter systems of the present invention utilize a high energy source to create a localized plasma in the catheter fluid that is retained within the inflatable balloon of the catheter, which in turn induces a high energy bubble inside the balloon to impart pressure onto and induce fractures in a treatment site, such as a calcified vascular lesion or a fibrous vascular lesion, at a treatment site within or adjacent to a blood vessel wall. Importantly, as described in detail herein, the catheter systems of the present invention include a fluid recirculation system that is configured to effectively monitor and/or control a temperature of the catheter fluid that is retained within the balloon so that the temperature of the catheter fluid within the balloon interior does not increase above safe levels, i.e. so that the temperature of the catheter fluid within the balloon interior is maintained within a safe, predetermined temperature range. Additionally, in some embodiments, the fluid recirculation system can further be configured to effectively monitor and/or control a pressure of the catheter fluid that is retained within the balloon so that the pressure is maintained at a generally desired pressure level, i.e. within a predetermined desired pressure range, during operation and use of the catheter system. Further, in certain embodiments, the fluid recirculation system can also be configured to filter out and/or remove unwanted microparticles from the catheter fluid so that the catheter system can more effectively and efficiently impart pressure onto and induce fractures in the treatment site. Still further, in some applications, the fluid recirculation system can be used to remove any undesired air bubbles that may have been formed within the catheter fluid within the balloon during the process of plasma generation, which may otherwise adversely impact the efficacy of the catheter system.

In particular, in various embodiments, the catheter systems can include a catheter configured to advance to the treatment site within or adjacent a blood vessel or heart valve. The catheter includes a catheter shaft, and a balloon that is coupled and/or secured to the catheter shaft. The balloons herein can include a balloon wall that defines a balloon interior. The balloons can be configured to receive a catheter fluid within the balloon interior to expand from a collapsed configuration suitable for advancing the catheter through a patient's vasculature, to an expanded configuration suitable for anchoring the catheter in position relative to the treatment site. The catheter systems also include one or more energy guides, e.g., light guides, disposed along the catheter shaft and within the balloon. Each light guide can be configured for generating pressure waves within the balloon for disrupting the vascular lesions. The catheter systems utilize energy from an energy source, e.g., light energy from a light source, to generate a plasma within the catheter fluid at or near a distal end of the light guide disposed within the balloon interior of the balloon located at the treatment site. The plasma formation can initiate a shockwave and can initiate the rapid formation of one or more bubbles that can rapidly expand to a maximum size and then dissipate through a cavitation event that can launch a shockwave upon collapse. The rapid expansion of the plasma-induced bubbles can generate one or more pressure waves within the catheter fluid retained within the balloon and thereby impart pressure waves upon the treatment site. In some embodiments, the light source can be configured to provide sub-millisecond pulses of light energy from the light source to initiate plasma formation in the catheter fluid within the balloon to cause rapid bubble formation and to impart pressure waves upon the balloon wall at the treatment site. Thus, the pressure waves can transfer mechanical energy through an incompressible catheter fluid to the treatment site to impart a fracture force on the intravascular lesion.

Additionally, as noted, the catheter systems further include the fluid recirculation system that can have any suitable design for purposes of monitoring and/or controlling the temperature of the catheter fluid within the balloon interior during use of the catheter system. Further, as noted, in some embodiments, the fluid recirculation system can also be configured for purposes of monitoring and/or controlling the pressure of the catheter fluid within the balloon interior during use of the catheter system. Still further, as provided herein, in certain embodiments, the fluid recirculation system can also be configured to filter out and/or remove undesired microparticles that may be generated within the catheter fluid during use of the catheter system. Yet further, in some applications, undesired air bubbles can be formed within the catheter fluid as energy from the energy guides contacts components within the balloon interior during the plasma generation process, and the recirculation of the catheter fluid with the fluid recirculation system can function to remove such undesired air bubbles from the catheter fluid.

As used herein, the terms "intravascular lesion", "vascular lesion" and "treatment site" are used interchangeably unless otherwise noted. As such, the intravascular lesions and/or the vascular lesions are sometimes referred to herein simply as "lesions".

Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention as illustrated in the accompanying drawings.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is appreciated that such a development effort might be complex and time-consuming, but would nevertheless be a routine undertaking of engineering for those of ordinary skill in the art having the benefit of this disclosure.

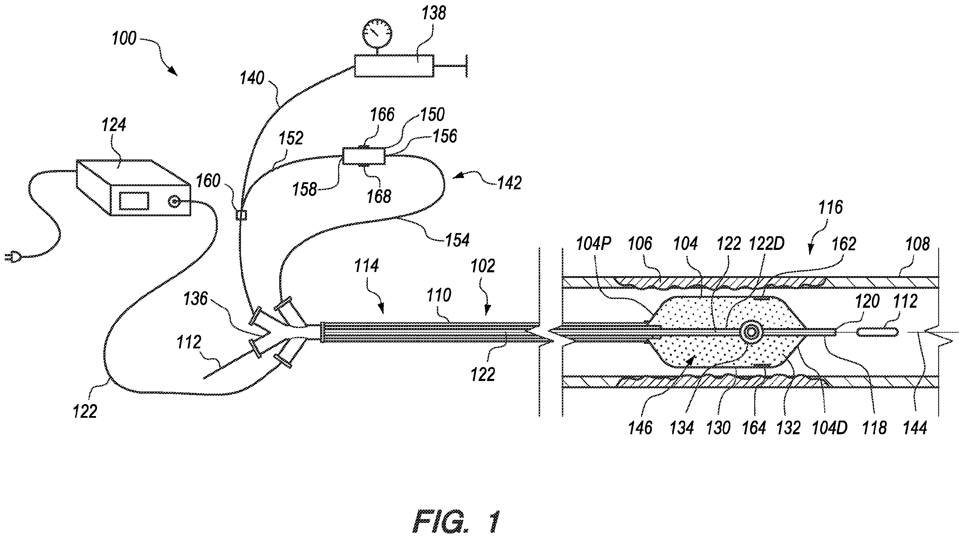

It is appreciated that the catheter systems disclosed herein can include many different forms. Referring now to Figure 1, a schematic cross-sectional view is shown of a catheter system 100 in accordance with various embodiments herein. As described herein, the catheter system 100 is suitable for imparting pressure to induce fractures in one or more treatment sites within or adjacent a vessel wall of a blood vessel or heart valve. In the embodiment illustrated in Figure 1, the catheter system 100 can include one or more of a catheter 102, one or more light guides 122, a light source 124, a source manifold 136, a fluid pump 138, and a fluid recirculation system 142.

The catheter 102 is configured to move to a treatment site 106 within or adjacent to a blood vessel 108 or heart valve. The treatment site 106 can include one or more vascular lesions such as calcified vascular lesions, for example. Additionally, or in the alternative, the treatment site 106 can include vascular lesions such as fibrous vascular lesions.

The catheter 102 can include an inflatable balloon 104 (sometimes referred to herein simply as a "balloon"), a catheter shaft 110 and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter shaft 110 can also include a guidewire lumen 118 which is configured to move over the guidewire 112. The catheter shaft 110 can further include an inflation lumen. In some embodiments, the catheter 102 can have a distal end opening 120 and can accommodate and be tracked over the guidewire 112 as the catheter 102 is moved and positioned at or near the treatment site 106.

The catheter shaft 110 of the catheter 102 can be coupled to the one or more light guides 122 (only one light guide is illustrated in Figure 1 for clarity) that are in optical communication with the light source 124. The light guide(s) 122 can be disposed along the catheter shaft 110 and within the balloon 104. In some embodiments, the light guide 122 can be an optical fiber and the light source 124 can be a laser. The light source 124 can be in optical communication with the light guide 122 at the proximal portion 114 of the catheter system 100.

In some embodiments, the catheter shaft 110 can be coupled to multiple light guides 122 such as a first light guide, a second light guide, a third light guide, etc., which can be disposed at any suitable positions about the guidewire lumen 118 and/or the catheter shaft 110. For example, in certain non-exclusive embodiments, two light guides 122 can be spaced apart by approximately 180 degrees about the circumference of the guidewire lumen 118 and/or the catheter shaft 110; three light guides 122 can be spaced apart by approximately 120 degrees about the circumference of the guidewire lumen 118 and/or the catheter shaft 110; or four light guides 122 can be spaced apart by approximately 90 degrees about the circumference of the guidewire lumen 118 and/or the catheter shaft 110. Still alternatively, multiple light guides 122 need not be uniformly spaced apart from one another about the circumference of the guidewire lumen 118 and/or the catheter shaft 110. More particularly, it is further appreciated that the light guides 122 described herein can be disposed uniformly or non-uniformly about the guidewire lumen 118 and/or the catheter shaft 110 to achieve the desired effect in the desired locations.

The balloon 104 can include a balloon wall 130 that defines a balloon interior 146, and can be inflated with a catheter fluid 132 to expand from a collapsed configuration suitable for advancing the catheter 102 through a patient's vasculature, to an expanded configuration suitable for anchoring the catheter 102 in position relative to the treatment site 106. Stated in another manner, when the balloon 104 is in the expanded configuration, the balloon wall 130 of the balloon 104 is configured to be positioned substantially adjacent to the treatment site 106. In some embodiments, the light source 124 of the catheter system 100 can be configured to provide sub-millisecond pulses of light from the light source 124, along the light guide 122, to a location within the balloon interior 146 of the balloon 104, thereby inducing plasma formation in the catheter fluid 132 within the balloon interior 146 of the balloon 104. The plasma formation causes rapid bubble formation, and imparts pressure waves upon the treatment site 106. Exemplary plasma-induced bubbles are shown as bubbles 134 in Figure 1.

It is appreciated that although the catheter systems 100 illustrated herein are generally described as including a light source 124 and one or more light guides 122A, the catheter system 100 can alternatively and equally include and/or utilize any suitable energy source and energy guides for purposes of generating the desired plasma in the balloon fluid 132 within the balloon interior 146. For example, in one non-exclusive alternative embodiment, the energy source 124 can be configured to provide high voltage pulses, and each energy guide 122A can include an electrode pair including spaced apart electrodes that extend into the balloon interior 146. In such embodiment, each pulse of high voltage is applied to the electrodes and forms an electrical arc across the electrodes, which, in turn, forms the pressure waves within the balloon fluid 132 that are utilized to provide the fracture force onto the treatment site 106. Still alternatively, the energy source 124 and/or the energy guides 122A can have another suitable design. Thus, although certain embodiments shown and described herein focus on "light sources" and "light guides", etc., it is understood that this is not intended to limit the disclosure herein to optical systems. Rather, it is recognized that other types of energy sources and energy guides can equally be utilized with the systems and methods provided herein, and that "light sources" and "light guides", etc. are provided by way of example and not by way of limitation, for ease in understanding the present disclosure.

The balloons 104 suitable for use in the catheter systems 100 described in detail herein include those that can be passed through the vasculature of a patient when in the collapsed configuration. In some embodiments, the balloons 104 herein are made from silicone. In other embodiments, the balloons 104 herein are made from polydimethylsiloxane (PDMS), polyurethane, polymers such as PEBAX^{™} material available from Arkema, which has a location at King of Prussia, Pennsylvania, USA, nylon, and the like. In some embodiments, the balloons 104 can include those having diameters ranging from one millimeter (mm) to 25 mm in diameter. In some embodiments, the balloons 104 can include those having diameters ranging from at least 1.5 mm to 12 mm in diameter. In some embodiments, the balloons 104 can include those having diameters ranging from at least one mm to five mm in diameter.

Additionally, in some embodiments, the balloons 104 herein can include those having a length ranging from at least five mm to 300 mm. More particularly, in some embodiments, the balloons 104 herein can include those having a length ranging from at least eight mm to 200 mm. It is appreciated that balloons 104 of greater length can be positioned adjacent to larger treatment sites 106, and, thus, may be usable for imparting pressure onto and inducing fractures in larger vascular lesions or multiple vascular lesions at precise locations within the treatment site 106. It is further appreciated that such longer balloons 104 can also be positioned adjacent to multiple treatment sites 106 at any given time.

Further, the balloons 104 herein can be inflated to desired inflation pressures of between approximately one atmosphere (atm) (101.325 kPa) to 70 atm (7092.75 kPa), i.e. a desired inflation pressure range. In some embodiments, the balloons 104 herein can be inflated to desired inflation pressures of from at least 20 atm (2026.5 kPa) to 70 atm (7092.75 kPa). In other embodiments, the balloons 104 herein can be inflated to desired inflation pressures of from at least six atm (607.95 kPa) to 20 atm (2026.5 kPa). In still other embodiments, the balloons 104 herein can be inflated to desired inflation pressures of from at least three atm (303.975 kPa) to 20 atm (2026.5 kPa). In yet other embodiments, the balloons 104 herein can be inflated to desired inflation pressures of from at least two atm (202.65 kPa) to ten atm (1013.25 kPa).

Still further, the balloons 104 herein can include those having various shapes, including, but not to be limited to, a conical shape, a square shape, a rectangular shape, a spherical shape, a conical/square shape, a conical/spherical shape, an extended spherical shape, an oval shape, a tapered shape, a bone shape, a stepped diameter shape, an offset shape, or a conical offset shape. In some embodiments, the balloons 104 herein can include a drug eluting coating or a drug eluting stent structure. The drug elution coating or drug eluting stent can include one or more therapeutic agents including anti-inflammatory agents, anti-neoplastic agents, anti-angiogenic agents, and the like.

The catheter fluid 132 can be a liquid or a gas. Exemplary catheter fluids 132 suitable for use herein can include, but are not limited to one or more of water, saline, contrast medium, fluorocarbons, perfluorocarbons, gases, such as carbon dioxide, and the like. In some embodiments, the catheter fluids 132 described can be used as base inflation fluids. In some embodiments, the catheter fluids 132 include a mixture of saline to contrast medium in a volume ratio of 50:50. In other embodiments, the catheter fluids 132 include a mixture of saline to contrast medium in a volume ratio of 25:75. In still other embodiments, the catheter fluids 132 include a mixture of saline to contrast medium in a volume ratio of 75:25. Additionally, the catheter fluids 132 suitable for use herein can be tailored on the basis of composition, viscosity, and the like in order to manipulate the rate of travel of the pressure waves therein. In certain embodiments, the catheter fluids 132 suitable for use herein are biocompatible. A volume of catheter fluid 132 can be tailored by the chosen light source 124 and the type of catheter fluid 132 used.

In some embodiments, the contrast agents used in the contrast media herein can include, but are not to be limited to, iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine based contrast agents can be used. Suitable non-iodine containing contrast agents can include gadolinium (III)-based contrast agents. Suitable fluorocarbon and perfluorocarbon agents can include, but are not to be limited to, agents such as the perfluorocarbon dodecafluoropentane (DDFP, C5F12).

Additionally, the catheter fluids 132 herein can include those that include absorptive agents that can selectively absorb light in the ultraviolet region (e.g., at least ten nanometers (nm) to 400 nm), the visible region (e.g., at least 400 nm to 780 nm), or the near-infrared region (e.g., at least 780 nm to 2.5 µm) of the electromagnetic spectrum. Suitable absorptive agents can include those with absorption maxima along the spectrum from at least ten nm to 2.5 µm. Alternatively, the catheter fluids 132 can include those that include absorptive agents that can selectively absorb light in the mid-infrared region (e.g., at least 2.5 µm to 15 µm), or the far-infrared region (e.g., at least 15 µm to one mm) of the electromagnetic spectrum. In various embodiments, the absorptive agent can be those that have an absorption maximum matched with the emission maximum of the laser used in the catheter system 100. In some embodiments, the absorptive agents used herein can be water soluble. In other embodiments, the absorptive agents used herein are not water soluble. In some embodiments, the absorptive agents used in the catheter fluids 132 herein can be tailored to match the peak emission of the light source 124. Various light sources 124 having emission wavelengths of at least ten nanometers to one millimeter are discussed elsewhere herein.

It is appreciated that the catheter system 100 disclosed herein can include any number of light guides 122 in optical communication with the light source 124 at the proximal portion 114, and with the catheter fluid 132 within the balloon interior 146 of the balloon 104 at the distal portion 116. For example, in some embodiments, the catheter system 100 can include from one light guide 122 to five light guides 122. In other embodiments, the catheter system 100 can include from five light guides 122 to fifteen light guides 122. In yet other embodiments, the catheter system 100 can include from ten light guides 122 to thirty light guides 122. Alternatively, in still other embodiments, the catheter system 100 can include greater than 30 light guides 122.

The light guides 122 herein can include an optical fiber or flexible light pipe. The light guides 122 herein can be thin and flexible and can allow light signals to be sent with very little loss of strength. The light guides 122 herein can include a core surrounded by a cladding about its circumference. In some embodiments, the core can be a cylindrical core or a partially cylindrical core. The core and cladding of the light guides 122 can be formed from one or more materials, including but not limited to one or more types of glass, silica, or one or more polymers. The light guides 122 may also include a protective coating, such as a polymer. It is appreciated that the index of refraction of the core will be greater than the index of refraction of the cladding.

Each light guide 122 can guide light along its length to a distal portion, i.e. a guide distal end 122D, having at least one optical window (not shown). The light guides 122 can create a light path as a portion of an optical network including the light source 124. The light path within the optical network allows light to travel from one part of the network to another. Both the optical fiber and the flexible light pipe can provide a light path within the optical networks herein.

Further, the light guides 122 herein can assume many configurations about and/or relative to the catheter shaft 110 of the catheters 102 described herein. In some embodiments, the light guides 122 can run parallel to the longitudinal axis 144 of the catheter shaft 110. In some embodiments, the light guides 122 can be physically coupled to the catheter shaft 110. In other embodiments, the light guides 122 can be disposed along a length of an outer diameter of the catheter shaft 110. In yet other embodiments, the light guides 122 herein can be disposed within one or more light guide lumens within the catheter shaft 110.

Additionally, it is further appreciated that the light guides 122 can be disposed at any suitable positions about the circumference of the guidewire lumen 118 and/or the catheter shaft 110, and the guide distal end of each of the light guides 122 can be disposed at any suitable longitudinal position relative to the length of the balloon 104 and/or relative to the length of the guidewire lumen 118.

Further, the light guides 122 herein can include one or more photoacoustic transducers (not shown in Figure 1), where each photoacoustic transducer can be in optical communication with the light guide 122 within which it is disposed. In some embodiments, the photoacoustic transducers can be in optical communication with the guide distal end 122D of the light guide 122. Additionally, in such embodiments, the photoacoustic transducers can have a shape that corresponds with and/or conforms to the guide distal end 122D of the light guide 122.

The photoacoustic transducer is configured to convert light energy into an acoustic wave at or near the guide distal end 122D of the light guide 122. It is appreciated that the direction of the acoustic wave can be tailored by changing an angle of the guide distal end 122D of the light guide 122.

It is further appreciated that the photoacoustic transducers disposed at the guide distal end 122D of the light guide 122 herein can assume the same shape as the guide distal end 122D of the light guide 122. For example, in certain non-exclusive embodiments, the photoacoustic transducer and/or the guide distal end 122D can have a conical shape, a convex shape, a concave shape, a bulbous shape, a square shape, a stepped shape, a half-circle shape, an ovoid shape, and the like. It is also appreciated that the light guide 122 can further include additional photoacoustic transducers disposed along one or more side surfaces of the length of the light guide 122.

The light guides 122 described herein can further include one or more diverting features or "diverters" (not shown in Figure 1) within the light guide 122 that are configured to direct light to exit the light guide 122 toward a side surface e.g., at or near the guide distal end 122D of the light guide 122, and toward the balloon wall 130. A diverting feature can include any feature of the system herein that diverts light from the light guide 122 away from its axial path toward a side surface of the light guide 122. Additionally, the light guides 122 can each include one or more light windows disposed along the longitudinal or axial surfaces of each light guide 122 and in optical communication with a diverting feature. Stated in another manner, the diverting features herein can be configured to direct light in the light guide 122 toward a side surface, e.g., at or near the guide distal end 122D, where the side surface is in optical communication with a light window. The light windows can include a portion of the light guide 122 that allows light to exit the light guide 122 from within the light guide 122, such as a portion of the light guide 122 lacking a cladding material on or about the light guide 122.

Examples of the diverting features suitable for use herein include a reflecting element, a refracting element, and a fiber diffuser. Additionally, the diverting features suitable for focusing light away from the tip of the light guides 122 herein can include, but are not to be limited to, those having a convex surface, a gradient-index (GRIN) lens, and a mirror focus lens. Upon contact with the diverting feature, the light is diverted within the light guide 122 to the photoacoustic transducer that is in optical communication with a side surface of the light guide 122. As noted, the photoacoustic transducer then converts light energy into an acoustic wave that extends away from the side surface of the light guide 122.

The light source 124 can have any suitable design. In certain embodiments, as noted above, the light source 124 can be configured to provide sub-millisecond pulses of light from the light source 124, which are directed along the light guides 122 to a location within the balloon 104, thereby inducing plasma formation in the catheter fluid 132 within the balloon interior 146 of the balloon 104. The plasma formation causes rapid bubble formation, and imparts pressure waves upon the treatment site 106. In such embodiments, the sub-millisecond pulses of light from the light source 124 can be delivered to the treatment site 106 at a frequency of between approximately one hertz (Hz) and 5000 Hz. In some embodiments, the sub-millisecond pulses of light from the light source 124 can be delivered to the treatment site 106 at a frequency of between approximately 30 Hz and 1000 Hz. In other embodiments, the sub-millisecond pulses of light from the light source 124 can be delivered to the treatment site 106 at a frequency of between approximately ten Hz and 100 Hz. In yet other embodiments, the sub-millisecond pulses of light from the light source 124 can be delivered to the treatment site 106 at a frequency of between approximately one Hz and 30 Hz. Alternatively, the sub-millisecond pulses of light can be delivered to the treatment site 106 at a frequency that can be greater than 5000 Hz.

The light sources 124 suitable for use herein can include various types of light sources including lasers and lamps. Alternatively, as noted above, the light sources 124, as referred to herein, can include any suitable type of energy source.

Suitable lasers can include short pulse lasers on the sub-millisecond timescale. In some embodiments, the light source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths and energy levels that can be employed to achieve plasma in the catheter fluid 132 of the catheters 102 described herein. In various embodiments, the pulse widths can include those falling within a range including from at least ten ns to 200 ns. In some embodiments, the pulse widths can include those falling within a range including from at least 20 ns to 100 ns. In other embodiments, the pulse widths can include those falling within a range including from at least one ns to 500 ns.

Additionally, exemplary nanosecond lasers can include those within the UV to IR spectrum, spanning wavelengths of about ten nanometers (nm) to one millimeter (mm). In some embodiments, the light sources 124 suitable for use in the catheter systems 100 herein can include those capable of producing light at wavelengths of from at least 750 nm to 2000 nm. In other embodiments, the light sources 124 can include those capable of producing light at wavelengths of from at least 700 nm to 3000 nm. In still other embodiments, the light sources 124 can include those capable of producing light at wavelengths of from at least 100 nm to ten micrometers (µm). Nanosecond lasers can include those having repetition rates of up to 200 kHz.

In some non-exclusive embodiments, the laser can include a Q-switched thulium:yttrium-aluminum-garnet (Tm:YAG) laser. In other non-exclusive embodiments, the laser can include a neodymium:yttrium-aluminum-garnet laser (Nd:YAG - emission maximum = 1064 nm), holmium:yttrium-aluminum-garnet laser (Ho:YAG - emission maximum = 2.1 µm), erbium:yttrium-aluminum-garnet laser (Er:YAG - emission maximum = 2.94 µm), excimer laser, helium-neon laser, carbon dioxide laser, as well as doped, pulsed, fiber lasers.

The catheter systems 100 disclosed herein can generate pressure waves having maximum pressures in the range of at least one megapascal (MPa) to 100 MPa. The maximum pressure generated by a particular catheter system 100 will depend on the light source 124, the absorbing material, the bubble expansion, the propagation medium, the balloon material, and other factors. In some embodiments, the catheter systems 100 herein can generate pressure waves having maximum pressures in the range of at least two MPa to 50 MPa. In other embodiments, the catheter systems 100 herein can generate pressure waves having maximum pressures in the range of at least two MPa to 30 MPa. In yet other embodiments, the catheter systems 100 herein can generate pressure waves having maximum pressures in the range of at least 15 MPa to 25 MPa.

The pressure waves described herein can be imparted upon the treatment site 106 from a distance within a range from at least 0.1 millimeters (mm) to 25 mm extending radially from the light guide 122 when the catheter 102 is placed at the treatment site 106. In some embodiments, the pressure waves can be imparted upon the treatment site 106 from a distance within a range from at least ten mm to 20 mm extending radially from the light guide 122 when the catheter 102 is placed at the treatment site 106. In other embodiments, the pressure waves can be imparted upon the treatment site 106 from a distance within a range from at least one mm to ten mm extending radially from the light guide 122 when the catheter 102 is placed at the treatment site 106. In yet other embodiments, the pressure waves can be imparted upon the treatment site 106 from a distance within a range from at least 1.5 mm to four mm extending radially from the light guide 122 when the catheter 102 is placed at the treatment site 106. In some embodiments, the pressure waves can be imparted upon the treatment site 106 from a range of at least two MPa to 30 MPa at a distance from 0.1 mm to ten mm. In some embodiments, the pressure waves can be imparted upon the treatment site 106 from a range of at least two MPa to 25 MPa at a distance from 0.1 mm to ten mm.

The source manifold 136 can be positioned at or near the proximal portion 114 of the catheter system 100. The source manifold 136 can include one or more proximal end openings that can receive the one or more light guides 122, the guidewire 112, a fluid inflow line 152 (sometimes referred to simply as an "inflow line"), and/or a fluid outflow line 154 (sometimes referred to simply as an "outflow line"). As illustrated in Figure 1, the inflow line 152 is in fluid communication with an inflation conduit 140 that is in fluid communication with the fluid pump 138. In this embodiment, the inflow line 152 is in fluid communication with the inflation conduit 140 via a fluid valve 160. Further, as illustrated and described herein, the inflow line 152 and the outflow line 154 can extend into the balloon interior 146 so as to form a portion of the fluid recirculation system 142. The fluid inflow line 152 includes an outlet port that is positioned within the balloon interior 146 such that the inflow line 152 is in fluid communication with the balloon interior 146, thus allowing the catheter fluid 132 to flow through the inflow line 152 and into the balloon interior 146 via the outlet port. Similarly, the fluid outflow line 154 includes an inlet port that is positioned within the balloon interior 146 such that the outflow line 154 is in fluid communication with the balloon interior 146, thus allowing the catheter fluid 132 to flow into the outflow line and out of the balloon interior 146 via the inlet port.

The catheter system 100 can also include the fluid pump 138 that is configured to inflate the balloon 104 with the catheter fluid 132 as needed. More particularly, as described in detail herein, during initial inflation of the balloon 104, the fluid valve 160 is in a first position so that the inflation conduit 140 is open to the inflow line 152. The fluid pump 138 pumps the catheter fluid 132 through the inflation conduit 140 and into the inflow line 152. The catheter fluid 132 is further directed through the inflow line 152 and into the balloon interior 146 of the balloon 104, i.e. via the outlet port of the inflow line 152. In such manner, the balloon 104 is inflated such that the catheter fluid 132 is within a desired fluid pressure range, i.e. the desired inflation pressure range, within the balloon interior 146 during use of the catheter system 100.

Additionally, as described in greater detail herein below, once the balloon 104 has been inflated with the catheter fluid 132 to the desired fluid pressure and/or the desired inflation pressure, the fluid valve 160 is moved to a second position whereby the inflation conduit 140 is no longer open into the inflow line 152. At this time, the light source 124 is controlled to direct light energy through the one or more light guides 122, so that the light energy is directed into the balloon interior 146, as described above. The light energy being directed into the catheter fluid 132 within the balloon interior 146 initiates plasma formation in the catheter fluid 132 within the balloon interior 146 to cause rapid bubble 134 formation and to impart desired pressure waves upon the balloon wall 130 at the treatment site 106. During this period of plasma formation and rapid bubble formation within the catheter fluid 132 within the balloon interior 146, the catheter fluid 132 can increase in temperature. As noted above, if left unchecked, the temperature of the catheter fluid 132 within the balloon interior 146 can thus increase to unsafe levels, which can damage the components of the catheter system 100, e.g., the balloon 104 where the integrity of the balloon wall 130 could potentially be compromised, and/or create undesired risks for the patient.

With the present disclosure, the fluid recirculation system 142 is configured to monitor and/or control a temperature and/or a pressure of the catheter fluid 132 within the balloon interior 146 so that the catheter fluid 132 does not increase to temperatures above safe levels. The design of the fluid recirculation system 142 can be varied to suit the requirements of the catheter system 100. As illustrated in Figure 1, the fluid recirculation system 142 includes a fluid circulator 150 that is in fluid communication to both the inflow line 152 and the outflow line 154, i.e. via a pump outlet 158 and a pump inlet 156, respectively. Additionally, as shown, in certain non-exclusive embodiments, the fluid recirculation system 142 can further include one or more of a fluid temperature sensor 162, a fluid pressure sensor 164, a fluid temperature controller 166, and a fluid pressure controller 168. Alternatively, the fluid recirculation system 142 can include more components or fewer components than what is specifically illustrated and described in relation to Figure 1. For example, in some non-exclusive alternative embodiments, the fluid recirculation system 142 can be configured for use without one or more of the fluid temperature sensor 162, the fluid pressure sensor 164, the fluid temperature controller 166, and/or the fluid pressure controller 168. Additionally, it is further appreciated that any of the fluid temperature sensor 162, the fluid pressure sensor 164, the fluid temperature controller 166 and the fluid pressure controller 168 can be positioned in any suitable locations within the catheter system 100, which may be different than what is specifically shown in Figure 1.

As an overview, after the balloon 104 has been inflated to the desired fluid pressure and/or the desired inflation pressure, the fluid valve 160 is moved to the second position, and the fluid circulator 150 is activated to selectively circulate the catheter fluid 132 through a cooling loop that effectively controls the temperature of the catheter fluid 132 within the balloon interior 146. More specifically, as the temperature of the catheter fluid 132 tends to rise during use of the catheter system 100, the fluid circulator 150 is activated to selectively pump out and/or remove catheter fluid 132 from the balloon interior 146, i.e. via the outflow line 154, circulate such catheter fluid 132 into and through the fluid circulator 150 where the temperature of the catheter fluid 132 can be lowered to a preferred fluid temperature, and pump catheter fluid 132 back into the balloon interior 146, i.e. via the inflow line 152. Stated in another manner, when activated by the user, the fluid circulator 150 circulates the catheter fluid 132 within the balloon interior 146 to actively cool the catheter fluid 132, and thus the balloon 104, during operation of the catheter system 100. With such design, the temperature of the catheter fluid 132 can be more effectively maintained at or near a desired fluid temperature within the balloon interior 146, even at times of plasma formation and bubble formation within the balloon interior 146. Moreover, the rate of the movement of the catheter fluid 132 into and out of the balloon interior 146 can be controlled, i.e. through use of the fluid recirculation system 142, e.g., the fluid circulator 150, so as to maintain the pressure of the catheter fluid 132 at or near the desired fluid pressure and/or desired inflation pressure level within the balloon interior 146.

As noted, in certain embodiments, the fluid recirculation system 142 can further include the fluid temperature sensor 162 that is configured to monitor and/or sense the temperature of the catheter fluid 132 within the balloon interior 146. Thus, the fluid recirculation system 142 is better able to control the selective activation of the fluid circulator 150 as described above. In particular, as the fluid temperature sensor 162 senses that the temperature of the catheter fluid 132 is beginning to rise within the balloon interior 146, the fluid temperature sensor 162 will generate a signal indicating that it is an appropriate time to activate the fluid circulator 150. Thus, upon activation of the fluid circulator 150, the temperature of the catheter fluid 132 within the balloon interior 146 can be more effectively controlled in the manner as described above. The fluid temperature sensor 162 can have any suitable design for purposes of monitoring and/or sensing the temperature of the catheter fluid 132 within the balloon interior 146. Alternatively, the fluid recirculation system 142 and/or the catheter system 100 can be designed without the fluid temperature sensor 162.

It is appreciated that the fluid temperature sensor 162 can be positioned in any appropriate location within the fluid recirculation system 142 in order to effectively monitor and/or sense the temperature of the catheter fluid 132 within the balloon interior 146. For example, in one embodiment, as shown in Figure 1, the fluid temperature sensor 162 can be positioned substantially within the balloon interior 146. Alternatively, the fluid temperature sensor 162 can be positioned in another suitable location. For example, in certain non-exclusive alternative embodiments, the fluid temperature sensor 162 can be positioned substantially adjacent to the balloon wall 130 outside of the balloon interior 146, within and/or adjacent to the fluid circulator 150, within and/or adjacent to the outflow line 154, and/or within and/or adjacent to the inflow line 152.

Additionally, as provided above, in some embodiments, the fluid recirculation system 142 can also include the fluid temperature controller 166. In such embodiments, the fluid temperature controller 166 can be configured to control the temperature of the catheter fluid 132 as the catheter fluid 132 is recirculated in a loop from within the balloon interior 146, toward and through the fluid circulator 150 (via the outflow line 154), and back into the balloon interior 146 (via the inflow line 152). The fluid temperature controller 166 can have any suitable design for purposes of actively controlling the temperature of the catheter fluid 132. For example, in one embodiment, the fluid temperature controller 166 can be a chiller that effectively cools the temperature of the catheter fluid 132 so that the catheter fluid 132 that has been heated within the balloon interior 146 is cooled to a predetermined lower temperature before being directed back into the balloon interior 146 via the fluid inflow line 152. As such, as the catheter fluid 132 re-enters the balloon interior 146 at a lower temperature, the overall temperature of the catheter fluid 132 within the balloon interior 146 can be effectively maintained at the desired temperature, e.g., 37 degrees Celsius in one non-exclusive application. Alternatively, the fluid temperature controller 166 can have another suitable design. Still alternatively, the fluid recirculation system 142 can be designed without the fluid temperature controller 166.

It is appreciated that the fluid temperature controller 166 can be positioned in any suitable location within the fluid recirculation system 142 in order to effectively control the temperature of the volume of the catheter fluid 132 that is being retained within the balloon interior 146. For example, in one non-exclusive embodiment, as shown in Figure 1, the fluid temperature controller 166 can be positioned substantially adjacent to the fluid circulator 150. Alternatively, the fluid temperature controller 166 can be positioned in another suitable location. For example, in certain non-exclusive alternative embodiments, the fluid temperature controller 166 can be positioned substantially adjacent to the fluid inflow line 152 and/or adjacent to the fluid outflow line 154.

Further, as noted above, in certain embodiments, the fluid recirculation system 142 can also include the fluid pressure sensor 164 that is configured to monitor and/or sense the fluid pressure and/or the inflation pressure of the catheter fluid 132 that is being retained within the balloon interior 146 of the balloon 104. As provided above, during initial inflation of the balloon 104, the fluid pump 138 is utilized to inflate the balloon 104 with the catheter fluid 132 to a desired fluid pressure and/or a desired inflation pressure. Additionally, it is desired that such inflation pressure within the balloon interior 146 be effectively maintained within a suitable range about the desired inflation pressure during operation of the catheter system 100. As such, the fluid pressure sensor 164 monitors and/or senses the inflation pressure within the balloon interior 146 to determine if the inflation pressure moves outside the suitable range about the desired inflation pressure. At such time, the fluid pressure sensor 164 can be configured to generate a signal indicating that it is an appropriate time to adjust the inflation pressure within the balloon interior 146 to a higher inflation pressure or a lower inflation pressure. In one embodiment, the inflation pressure can be adjusted through adjusting the rate of the catheter fluid 132 being moved out of the balloon interior 146 and/or back into the balloon interior 146 with the fluid circulator 150. The fluid pressure sensor 164 can have any suitable design for purposes of monitoring and/or sensing the inflation pressure of the catheter fluid 132 within the balloon interior 146. Alternatively, the fluid recirculation system 142 can be designed without the fluid pressure sensor 164.

It is appreciated that the fluid pressure sensor 164 can be positioned in any appropriate location within the fluid recirculation system 142 in order to effectively monitor and/or sense the inflation pressure of the catheter fluid 132 within the balloon interior 146. For example, in one embodiment, as shown in Figure 1, the fluid pressure sensor 164 can be positioned substantially within the balloon interior 146. Alternatively, the fluid pressure sensor 164 can be positioned in another suitable location. For example, in certain non-exclusive alternative embodiments, the fluid pressure sensor 164 can be positioned substantially adjacent to the balloon wall 130 outside of the balloon interior 146, within and/or adjacent to the fluid circulator 150, within and/or adjacent to the outflow line 154, and/or within and/or adjacent to the inflow line 152.

Still further, as provided above, in some embodiments, the fluid recirculation system 142 can further include the fluid pressure controller 168. In such embodiments, the fluid pressure controller 168 can be configured to control the inflation pressure of the catheter fluid 132 within the balloon interior 146 as the catheter fluid 132 is recirculated in a loop from within the balloon interior 146, toward and through the fluid circulator 150 (via the outflow line 154), and back into the balloon interior 146 (via the inflow line 152). The fluid pressure controller 168 can have any suitable design for purposes of actively controlling the inflation pressure of the catheter fluid 132 within the balloon interior 146. Alternatively, the fluid recirculation system 142 can be designed without the fluid pressure controller 168. Whether or not the fluid recirculation system 142 includes the fluid pressure controller 168, it is understood that it is generally preferable that the positive pressure of the catheter fluid 132 being pumped into the balloon 104 by the fluid circulator 150 via the fluid inflow line 152 perfectly balances the negative pressure of the catheter fluid 132 being removed/pumped/sucked out of the balloon 104 by the fluid circulator 150 via the fluid outflow line 154 to maintain the desired inflation pressure within the balloon interior 146.

It is appreciated that the fluid pressure controller 168 can be positioned in any suitable location within the fluid recirculation system 142 in order to effectively control the inflation pressure of the catheter fluid 132 that is being retained within the balloon interior 146. For example, in one non-exclusive embodiment, as shown in Figure 1, the fluid pressure controller 168 can be positioned substantially adjacent to the fluid circulator 150. Alternatively, the fluid pressure controller 168 can be positioned in another suitable location. For example, in certain non-exclusive alternative embodiments, the fluid pressure controller 168 can be positioned substantially adjacent to the fluid inflow line 152 and/or adjacent to the fluid outflow line 154.

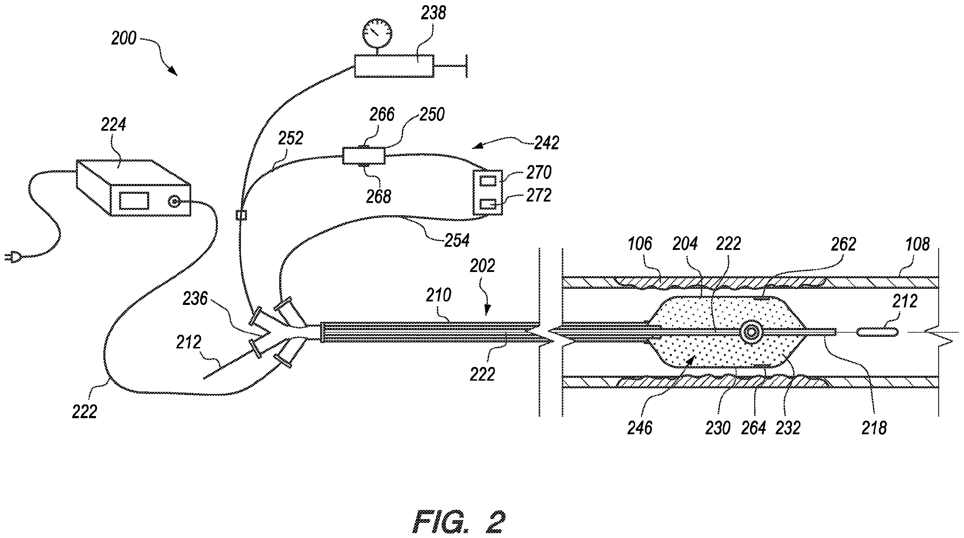

Figure 2 is a schematic cross-sectional view of another embodiment of the catheter system another embodiment of the fluid recirculation system 242. As illustrated, the catheter system 200 is substantially similar in design and operation to the catheter system 100 illustrated and described in detail above. For example, the catheter system 200 again includes a catheter 202, including a balloon 204, a catheter shaft 210, a guidewire 212 and a guidewire lumen 218, one or more light guides 222, a light source 224, a source manifold 236, and a fluid pump 238 that are substantially similar in design and operation to such components illustrated and described above. As in the previous embodiment, the balloon 204 is configured to be positioned at a treatment site 106 within or adjacent to a blood vessel 108 or heart valve that includes one or more vascular lesions. Additionally, the balloon 204 again includes a balloon wall 230 that defines a balloon interior 246; and the fluid pump 238 is again configured to pump catheter fluid 232 into the balloon interior 246 to expand the balloon 204 from a collapsed configuration to an expanded configuration. Further, the light source 224 can again be configured to provide sub-millisecond pulses of light from the light source 224, along the light guide 222, to a location within the balloon interior 246 of the balloon 204, thereby inducing plasma formation in the catheter fluid 232 within the balloon interior 246 of the balloon 204. Thus, such features and components will not again be described in detail in relation to the embodiments shown in Figure 2.

However, in the embodiment illustrated in Figure 2, the fluid recirculation system 242 has a slightly different design than what was shown in the previous embodiment. As with the previous embodiment, the fluid recirculation system 242 is again configured to monitor and/or control a temperature and/or a pressure of the catheter fluid 232 within the balloon interior 246 so that the catheter fluid 232 does not increase to temperatures above safe levels. As such, the fluid recirculation system 242 again includes a fluid circulator 250 that is in fluid communication to both the fluid inflow line 252 and the fluid outflow line 254. When activated, the fluid circulator 250 selectively circulates the catheter fluid 232 through a loop whereby the catheter fluid 232 is pumped out and/or removed from the balloon interior 246 via the fluid outflow line 254, and subsequently pumped back into the balloon interior 246 via the fluid inflow line 252. Thus, as with the previous embodiment, the recirculation of the catheter fluid 232 can function to control the temperature and/or the pressure of the catheter fluid 232 within the balloon interior.

Additionally, as with the previous embodiment, the fluid recirculation system 242 can again include one or more of a fluid temperature sensor 262, a fluid pressure sensor 264, a fluid temperature controller 266, and a fluid pressure controller 268 that can aid in the process of monitoring and/or controlling the temperature and/or the pressure of the catheter fluid 232 within the balloon interior. Alternatively, as above, the fluid recirculation system 242 can be configured without one or more of the fluid temperature sensor 262, the fluid pressure sensor 264, the fluid temperature controller, and/or the fluid pressure controller 268.

However, in the embodiment shown in Figure 2, the fluid recirculation system 242 is further configured to filter out and/or remove any undesired microparticles that may have been formed within the catheter fluid 232. As described above, when energy is emitted from the energy guide 222 into the catheter fluid 232 within the balloon interior 246, the energy can hit a plasma target or other structures within the balloon interior, which can cause microparticles to get into the catheter fluid 232. The microparticles being formed within the catheter fluid 232 can cause the plasma to be less concentrated and more distributed within the catheter fluid 232, which can have an adverse impact of the effectiveness and/or the efficiency of the catheter system 200. More specifically, during use of the catheter system 200, it is desired to have concentrated plasma bursts that are generated within the catheter fluid 232 due to the energy from the energy source 224 being directed through the energy guide 222 and into the catheter fluid 232 within the balloon interior 246.

Accordingly, in certain embodiments, as shown in Figure 2, the fluid recirculation system 242 can further include a filtration system 270 that is in fluid communication with the recirculation loop of the catheter fluid 232 through the fluid circulator 250. With such design, the catheter fluid 232 will also pass through the filtration system 270 during movement of the catheter fluid 232 through the recirculation loop. The filtration system 270 is thus configured to filter out and/or remove any undesired microparticles that have been formed within the catheter fluid 232.

It is appreciated that the filtration system 270 can have any suitable design for purposes of filtering out and/or removing undesired microparticles from within the catheter fluid 232. For example, in some embodiments, the filtration system 270 can include one or more filters 272 (two are shown in Figure 2) that are specifically designed to filter out and remove microparticles of a certain size from the catheter fluid 232 that passes through the filtration system 270. Alternatively, the filtration system 270 can have another suitable design.

Additionally, it is further appreciated that the filtration system 270 can be positioned in any suitable location within the fluid recirculation system 242, i.e. within the recirculation loop of the catheter fluid 232. For example, in one non-exclusive embodiment, as shown in Figure 2, the filtration system 270 can be positioned within and/or along the fluid outflow line 254. With such design, the undesired microparticles can be removed from the catheter fluid 232 prior to the catheter fluid 232 passing through the fluid circulator 250. Thus, the microparticles can be inhibited from having any adverse impacts on the operation of the fluid circulator 250 itself. Alternatively, the filtration system 270 can be positioned in another suitable location within the recirculation loop. For example, in certain non-exclusive alternative embodiments, the filtration system 270 can be positioned within and/or along the fluid inflow line 252, substantially adjacent to the fluid circulator 250, or at another suitable location.

In summary, it is appreciated that the catheter systems and methods described in detail herein can provide one or more advantages, such as:
(1) monitoring and/or controlling the temperature of the catheter fluid within the balloon interior of the balloon during use of the catheter system;
(2) monitoring and/or controlling the pressure of the catheter fluid within the balloon interior of the balloon during use of the catheter system;
(3) filtering out and/or removing any undesired microparticles that may be formed within the catheter fluid during use of the catheter system; and
(4) recirculating the catheter fluid to remove any undesired air bubbles that may have formed within the catheter fluid during plasma generation within the catheter fluid.

It should be noted that, as used in this specification and the appended claims, the singular forms "a," "an," and "the" include plural referents unless the content and/or context clearly dictates otherwise. It should also be noted that the term "or" is generally employed in its sense including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the present detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope herein.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions and sub-combinations thereof.

## Claims

1. A catheter system (100) for treating a treatment site (106) within or adjacent to a vessel wall or heart valve, the catheter system (100) comprising:
a balloon (104) including a balloon wall (130) that defines a balloon interior (146), the balloon (104) being configured to selectively retain a catheter fluid (132) within the balloon interior (146);
a fluid pump (138) that is configured to pump the catheter fluid (132) into the balloon interior (146) via an inflation conduit (140) to expand the balloon (104) from a collapsed configuration so that the balloon is configured to be moved relative to the treatment site (106), to an expanded configuration suitable for anchoring the balloon (104) in position relative to the treatment site (106);
an energy guide (122) that is configured to guide energy into the balloon interior (146) to generate a plasma within the catheter fluid (132) that is retained within the balloon interior (146); and
a fluid circulator (150) that is in fluid communication with the balloon interior (146), the fluid circulator (150) being configured to selectively recirculate the catheter fluid (132) in a recirculation loop that includes the balloon interior (146) and the fluid circulator (150) as the catheter fluid (132) is recirculated out of and back into the balloon interior (146) during use of the catheter system (100).

2. The catheter system (100) of claim 1 wherein the fluid circulator (150) selectively recirculates the catheter fluid (132) in the recirculation loop out of the balloon interior (146), toward and through the fluid circulator (150), and back into the balloon interior (146) during use of the catheter system (100).

3. The catheter system (100) of any one of claims 1 or 2 wherein the fluid circulator (150) is configured so that a temperature of the catheter fluid (132) within the balloon interior (146) is maintained within a predetermined temperature range during use of the catheter system (100).

4. The catheter system (100) of claim 3 further comprising a fluid temperature sensor (162) that is configured to sense the temperature of the catheter fluid (132) within the balloon interior (146); and wherein the fluid temperature sensor (162) is configured to generate a signal to activate the fluid circulator (150) if the temperature of the catheter fluid (132) is beginning to rise within the balloon interior (146).

5. The catheter system (100) of claim 4 further comprising a fluid temperature controller (166) that controls the temperature of the catheter fluid (132) as the catheter fluid (132) is being circulated by the fluid circulator (150).

6. The catheter system (100) of any of claims 1-5 wherein the fluid circulator (150) is configured so that a pressure of the catheter fluid (132) within the balloon interior (146) is maintained within a predetermined pressure range during use of the catheter system (100).

7. The catheter system (100) of claim 6 further comprising a fluid pressure sensor (164) that senses a pressure of the catheter fluid (132) within the balloon interior (146); and wherein the fluid pressure sensor (164) is configured to generate a signal when the pressure of the catheter fluid (132) within the balloon interior (146) falls outside the predetermined pressure range, the signal indicating when to selectively activate the fluid circulator (150) to adjust at least one of (i) a first rate at which the circulation fluid is being pumped out of the balloon interior (146) by the fluid circulator (150), and (ii) a second rate at which the circulation fluid is being pumped back into the balloon interior (146) by the fluid circulator (150).

8. The catheter system (100) of claim 7 further comprising a fluid pressure controller (168) that controls the pressure of the catheter fluid (132) as the catheter fluid (132) is being circulated by the fluid circulator (150).

9. The catheter system (100) of any of claims 1-8 further comprising a fluid inflow line (152) and a fluid outflow line (154) that are in fluid communication with the fluid circulator (150), the fluid circulator (150) circulating the catheter fluid (132) out of the balloon interior (146) via the fluid outflow line (154) and back into the balloon interior (146) via the fluid inflow line (152).

10. The catheter system (100) of claim 9 wherein the fluid pump (138) is in fluid communication with the fluid inflow line (152) via a fluid valve (160).

11. The catheter system (100) of claim 10 wherein, when the fluid valve (160) is in a first position, the inflation conduit (140) is open to the fluid inflow line (152), and the fluid pump (138) is configured to pump the catheter fluid (132) through the inflation conduit (140), into and through the fluid inflow line (152), and then into the balloon interior (146).

12. The catheter system (100) of claim 11 wherein, when the fluid valve (160) is in a second position, the inflation conduit (140) is not open into the fluid inflow line (152).

13. The catheter system (100) of any of claims 1-12 further comprising an energy source (224) that generates energy; and wherein the energy guide (222) receives the energy from the energy source (224) and guides the energy from the energy source (224) along the energy guide (222) into the balloon interior (146) to generate plasma in the catheter fluid (132) within the balloon interior (146), the plasma generation causing rapid bubble formation and imparting pressure waves upon the balloon wall (130) adjacent to the treatment site (106).

14. The catheter system (100) of claim 13 wherein the energy guide (222) includes an optical fiber; and wherein the energy source (124) includes a laser.

15. The catheter system (100) of any of claims 1-14 further comprising a filtration system (270) that removes microparticles from the catheter fluid (132) as the catheter fluid (132) is being circulated by the fluid circulator (150) through the recirculation loop.

## Patentansprüche

1. Kathetersystem (100) zur Behandlung einer Behandlungsstelle (106) in oder neben einer Gefäßwand oder einer Herzklappe, wobei das Kathetersystem (100) umfasst:
einen Ballon (104), der eine Ballonwand (130) umfasst, die einen Balloninnenraum (146) definiert, wobei der Ballon (104) ausgelegt ist, um ein Katheterfluid (132) selektiv im Balloninnenraum (146) zu halten;
eine Fluidpumpe (138), die ausgelegt ist, um das Katheterfluid (132) über eine Aufblasleitung (140) in den Balloninnenraum (146) zu pumpen, um den Ballon (104) von einer zusammengefallenen Konfiguration, in welcher der Ballon ausgelegt ist, um relativ zur Behandlungsstelle (106) bewegt zu werden, zu einer ausgedehnten Konfiguration auszudehnen, die zur Verankerung des Ballons (104) in einer Position relativ zur Behandlungsstelle (106) geeignet ist;
einen Energieleiter (122), der ausgelegt ist, um Energie in den Balloninnenraum (146) zu leiten, um ein Plasma im Katheterfluid (132) zu erzeugen, das im Balloninnenraum (146) gehalten wird; und
einen Fluidzirkulator (150), der in Fluidkommunikation mit dem Balloninnenraum (146) ist, wobei der Fluidzirkulator (150) ausgelegt ist, um das Katheterfluid (132) selektiv in einer Rezirkulationsschleife zu rezirkulieren, die den Balloninnenraum (146) und den Fluidzirkulator (150) umfasst, wenn das Katheterfluid (132) während der Verwendung des Kathetersystems (100) aus dem Balloninnenraum (146) heraus und in diesen hinein rezirkuliert wird.

2. Kathetersystem (100) nach Anspruch 1, wobei der Fluidzirkulator (150) das Katheterfluid (132) während der Verwendung des Kathetersystems (100) selektiv in der Rezirkulationsschleife aus dem Balloninnenraum (146), zum Fluidzirkulator (150) und durch diesen hindurch und zurück in den Balloninnenraum (146) rezirkuliert.

3. Kathetersystem (100) nach einem der Ansprüche 1 oder 2, wobei der Fluidzirkulator (150) so ausgelegt ist, dass die Temperatur des Katheterfluids (132) im Balloninnenraum (146) während der Verwendung des Kathetersystems (100) innerhalb eines vorbestimmten Temperaturbereichs gehalten wird.

4. Kathetersystem (100) nach Anspruch 3, das außerdem einen Fluidtemperatursensor (162) umfasst, der ausgelegt ist, die Temperatur des Katheterfluids (132) im Balloninnenraum (146) abzufühlen; und wobei der Fluidtemperatursensor (162) ausgelegt ist, um ein Signal zur Aktivierung des Fluidzirkulators (150) zu erzeugen, wenn die Temperatur des Katheterfluids (132) im Balloninnenraum (146) zu steigen beginnt.

5. Kathetersystem (100) nach Anspruch 4, das außerdem einen Fluidtemperaturregler (166) umfasst, der die Temperatur des Katheterfluids (132) regelt, während das Katheterfluid (132) vom Fluidzirkulator (150) zirkuliert wird.

6. Kathetersystem (100) nach einem der Ansprüche 1-5, wobei der Fluidzirkulator (150) so ausgelegt ist, dass der Druck des Katheterfluids (132) im Balloninnenraum (146) während der Verwendung des Kathetersystems (100) in einem vorbestimmten Druckbereich gehalten wird.

7. Kathetersystem (100) nach Anspruch 6, das außerdem einen Fluiddrucksensor (164) umfasst, der den Druck des Katheterfluids (132) im Balloninnenraum (146) abfühlt; und wobei der Fluiddrucksensor (164) ausgelegt ist, um ein Signal zu erzeugen, wenn der Druck des Katheterfluids (132) im Balloninnenraum (146) den vorbestimmten Druckbereich verlässt, wobei das Signal anzeigt, wann der Fluidzirkulator (150) selektiv aktiviert werden soll, um zumindest eines aus (i) einer ersten Rate, mit der das Zirkulationsfluid vom Fluidzirkulator (150) aus dem Balloninnenraum (146) gepumpt wird, und (ii) einer zweiten Rate, mit der das Zirkulationsfluid vom Fluidzirkulator (150) zurück in den Balloninnenraum (146) gepumpt wird, anzupassen.

8. Kathetersystem (100) nach Anspruch 7, das weiters einen Fluiddruckregler (168) umfasst, der den Druck des Katheterfluids (132) regelt, während das Katheterfluid (132) vom Fluidzirkulator (150) zirkuliert wird.

9. Kathetersystem (100) nach einem der Ansprüche 1-8, das außerdem eine Fluidzulaufleitung (152) und eine Fluidabflussleitung (154) umfasst, die in Fluidkommunikation mit dem Fluidzirkulator (150) sind, wobei der Fluidzirkulator (150) das Katheterfluid (132) über die Fluidabflussleitung (154) aus dem Balloninnenraum (146) und über die Fluidzulaufleitung (152) zurück in den Balloninnenraum (146) zirkuliert.

10. Kathetersystem (100) nach Anspruch 9, wobei die Fluidpumpe (138) über ein Fluidventil (160) in Fluidkommunikation mit der Fluidzulaufleitung (152) ist.

11. Kathetersystem (100) nach Anspruch 10, wobei, wenn das Fluidventil (160) in einer ersten Stellung ist, die Aufblasleitung (140) zur Fluidzulaufleitung (152) offen ist, und die Fluidpumpe (138) ausgelegt ist, um das Katheterfluid (132) durch die Aufblasleitung (140), in und durch die Fluidzulaufleitung (152) und dann in den Balloninnenraum (146) zu pumpen.

12. Kathetersystem (100) nach Anspruch 11, wobei, wenn das Fluidventil (160) in einer zweiten Stellung ist, die Aufblasleitung (140) nicht offen zur Fluidzulaufleitung (152) ist.

13. Kathetersystem (100) nach einem der Ansprüche 1-12, das außerdem eine Energiequelle (224) umfasst, die Energie erzeugt; und wobei der Energieleiter (222) die Energie von der Energiequelle (224) empfängt und die Energie von der Energiequelle (224) entlang des Energieleiters (222) in den Balloninnenraum (146) leitet, um Plasma im Katheterfluid (132) im Balloninnenraum (146) zu erzeugen, wobei die Plasmaerzeugung eine rasche Blasenbildung bewirkt und Druckwellen auf die Ballonwand (130) neben der Behandlungsstelle (106) erzeugt.

14. Kathetersystem (100) nach Anspruch 13, wobei der Energieleiter (222) eine optische Faser umfasst; und wobei die Energiequelle (124) einen Laser umfasst.

15. Kathetersystem (100) nach einem der Ansprüche 1-14, das außerdem ein Filtrationssystem (270) umfasst, das Mikropartikel aus dem Katheterfluid (132) aufnimmt, währen das Katheterfluid (132) vom Fluidzirkulator (150) durch die Rezirkulationsschleife zirkuliert wird.

## Revendications

1. Système de cathéter (100) pour traiter un site de traitement (106) au sein d'une paroi de vaisseau ou d'une valve cardiaque ou adjacente à celle-ci, le système de cathéter (100) comprenant :
un ballonnet (104) incluant une paroi de ballonnet (130) qui définit un intérieur de ballonnet (146), le ballonnet (104) étant configuré pour retenir sélectivement un fluide de cathéter (132) au sein de l'intérieur de ballonnet (146) ;
une pompe à fluide (138) qui est configurée pour pomper le fluide de cathéter (132) dans l'intérieur de ballonnet (146) via un conduit de gonflage (140) afin de dilater le ballonnet (104) d'une configuration repliée de sorte que le ballonnet est configuré pour être mis en mouvement par rapport au site de traitement (106), à une configuration dilatée convenant pour ancrer le ballonnet (104) en position par rapport au site de traitement (106) ;
un guide d'énergie (122) qui est configuré pour guider l'énergie dans l'intérieur de ballonnet (146) afin de générer un plasma dans le fluide de cathéter (132) qui est retenu au sein de l'intérieur de ballonnet (146) ; et
un circulateur de fluide (150) qui est en communication de fluide avec l'intérieur de ballonnet (146), le circulateur de fluide (150) étant configuré pour faire recirculer sélectivement le fluide de cathéter (132) dans une boucle de recirculation qui inclut l'intérieur de ballonnet (146) et le circulateur de fluide (150) à mesure que le fluide de cathéter (132) est remis en circulation hors de l'intérieur de ballonnet (146) et de retour dans celui-ci pendant l'utilisation du système de cathéter (100).

2. Système de cathéter (100) selon la revendication 1, dans lequel le circulateur de fluide (150) fait recirculer sélectivement le fluide de cathéter (132) dans la boucle de recirculation hors de l'intérieur de ballonnet (146), vers et à travers le circulateur de fluide (150), et de retour dans l'intérieur de ballonnet (146) pendant l'utilisation du système de cathéter (100).

3. Système de cathéter (100) selon l'une quelconque des revendications 1 ou 2, dans lequel le circulateur de fluide (150) est configuré de sorte qu'une température du fluide de cathéter (132) au sein de l'intérieur de ballonnet (146) est maintenue dans une plage de température prédéterminée pendant l'utilisation du système de cathéter (100).

4. Système de cathéter (100) selon la revendication 3, comprenant en outre un capteur de température de fluide (162) qui est configuré pour capter la température du fluide de cathéter (132) au sein de l'intérieur de ballonnet (146) ; et dans lequel le capteur de température de fluide (162) est configuré pour générer un signal afin d'activer le circulateur de fluide (150) si la température du fluide de cathéter (132) commence à monter au sein de l'intérieur de ballonnet (146).

5. Système de cathéter (100) selon la revendication 4, comprenant en outre un régulateur de température de fluide (166) qui régule la température du fluide de cathéter (132) lorsque le fluide de cathéter (132) est mis en circulation par le circulateur de fluide (150).

6. Système de cathéter (100) selon l'une quelconque des revendications 1 à 5, dans lequel le circulateur de fluide (150) est configuré de sorte qu'une pression du fluide de cathéter (132) au sein de l'intérieur de ballonnet (146) est maintenue dans une plage de pression prédéterminée pendant l'utilisation du système de cathéter (100).

7. Système de cathéter (100) selon la revendication 6, comprenant en outre un capteur de pression de fluide (164) qui capte une pression du fluide de cathéter (132) au sein de l'intérieur de ballonnet (146) ; et dans lequel le capteur de pression de fluide (164) est configuré pour générer un signal lorsque la pression du fluide de cathéter (132) au sein de l'intérieur de ballonnet (146) tombe en dehors d'une plage de pression prédéterminée, le signal indiquant quand activer sélectivement le circulateur de fluide (150) afin d'ajuster au moins une parmi : (i) une première vitesse à laquelle le fluide de circulation est pompé hors de l'intérieur de ballonnet (146) par le circulateur de fluide (150), et (ii) une seconde vitesse à laquelle le fluide de circulation est pompé de retour dans l'intérieur de ballonnet (146) par le circulateur de fluide (150).

8. Système de cathéter (100) selon la revendication 7, comprenant en outre un régulateur de pression de fluide (168) qui régule la pression du fluide de cathéter (132) lorsque le fluide de cathéter (132) est mis en circulation par le circulateur de fluide (150).

9. Système de cathéter (100) selon l'une quelconque des revendications 1 à 8, comprenant en outre une ligne d'arrivée de fluide (152) et une ligne de sortie de fluide (154) qui sont en communication de fluide avec le circulateur de fluide (150), le circulateur de fluide (150) faisant circuler le fluide de cathéter (132) hors de l'intérieur de ballonnet (146) via la ligne de sortie de fluide (154) et de retour dans l'intérieur de ballonnet (146) via la ligne d'arrivée de fluide (152).

10. Système de cathéter (100) selon la revendication 9, dans lequel la pompe à fluide (138) est en communication de fluide avec la ligne d'arrivée de fluide (152) via une vanne de fluide (160).

11. Système de cathéter (100) selon la revendication 10 dans lequel, lorsque la vanne de fluide (160) est dans une première position, le conduit de gonflage (140) débouche sur la ligne d'arrivée de fluide (152), et la pompe à fluide (138) est configurée pour pomper le fluide de cathéter (132) à travers le conduit de gonflage (140), dans et à travers la ligne d'arrivée de fluide (152), puis dans l'intérieur de ballonnet (146).

12. Système de cathéter (100) selon la revendication 11 dans lequel, lorsque la vanne de fluide (160) est dans une seconde position, le conduit de gonflage (140) ne débouche pas sur la ligne d'arrivée de fluide (152).

13. Système de cathéter (100) selon l'une quelconque des revendications 1 à 12, comprenant en outre une source d'énergie (224) qui génère de l'énergie ; et dans lequel le guide d'énergie (222) reçoit l'énergie de la source d'énergie (224) et guide l'énergie de la source d'énergie (224) le long du guide d'énergie (222) dans l'intérieur de ballonnet (146) afin de générer du plasma dans le fluide de cathéter (132) au sein de l'intérieur de ballonnet (146), la génération de plasma entraînant la formation rapide de bulles et communiquant des ondes de pression sur la paroi de ballonnet (130) adjacente au site de traitement (106).

14. Système de cathéter (100) selon la revendication 13, dans lequel le guide d'énergie (222) inclut une fibre optique ; et dans lequel la source d'énergie (124) inclut un laser.

15. Système de cathéter (100) selon l'une quelconque des revendications 1 à 14, comprenant en outre un système de filtration (270) qui élimine des microparticules du fluide de cathéter (132) lorsque le fluide de cathéter (132) est mis en circulation par le circulateur de fluide (150) à travers la boucle de recirculation.
